# EUROPEAN PATENT APPLICATION

(11) **EP 2 210 505 A1**
(43) Date of publication of application: **28.07.2010**
(21) Application number: 09151383.8
(22) Date of filing: 27.01.2009
(51) Int. Cl.: A23L 1/30, A61K 36/00, A61K 31/216

(54) **Composition comprising caftaric acid and/or derivatives thereof**

(71) Applicant: Nestec S.A., 1800 Vevey (CH)
(72) Inventor: Bel-Rhlid, Rachid, 1073 Savigny (CH); Crespy, Vanessa, 1800 Vevey (CH); Raab, Thomas, 1091 Grandvaux (CH); Page-Zoerkler, Nicole, 1066 Epalinges (CH); Fumeaux, René, 1807 Blonay (CH); Marin-Kuan, Maricel, 1012 Lausanne (CH); Piguet, Dominique, 1318 Pompaples (CH)
(74) Representative: Marquardt, Ulf

(57) **Abstract**

The present invention relates generally to the field of food and drinks. In particular, for example, a composition is provided that allows to provide tartaric and/or caffeic acid to a subject. One embodiment of the present invention is a composition comprising an ingredient containing caftaric acid and/or derivatives thereof, and a micro-organism and/or an enzyme capable of hydrolysing caftaric acid and/or derivatives thereof to generate tartaric and/or caffeic acid.

## Description

The present invention relates generally to the field of food and drinks. In particular, for example, a composition is provided that allows to provide tartaric and/or caffeic acid to a subject. One embodiment of the present invention is a composition comprising an ingredient containing caftaric acid and/or derivatives thereof, and a micro-organism and/or an enzyme capable of hydrolysing caftaric acid and/or derivatives thereof to generate tartaric and/or caffeic acid.

Caffeic Acid (CA) was shown to have tumor-shrinking properties. The subcutaneous and oral administrations of CA significantly reduced liver metastasis. These results confirm the therapeutic potential of CA and suggest that the anti-metastatic and antitumor effects of CA are mediated through the selective suppression of MMP-9 enzyme activity and transcriptional down-regulation by the dual inhibition of NF-κB as well as MMP-9 catalytic activity. [Tae-Wook Chung, et al., FASEB Journal. 2004:18:1670-1681]

The caffeic acid derivative, caffeic acid phenethyl ester (CAPE), is known to have antimitogenic, anticarcinogenic, anti-inflammatory, and immunomodulatory properties. [Natarajan et al., Proc Natl Acad Sci U S A. 1996 August 20; 93(17): 9090-9095] CAPE also suppresses acute immune and inflammatory responses [ Orban et al.,NeurolmmunoModulation 2000;7:99-105]

The anti-inflammatory and anti-cancer property has also been shown to protect skin cells when exposed to ultraviolet (UV) radiation, in particular UVC radiation [NERADIL, R. et al., Folia Biologica (Praha). 2003; 49:197-202] and UVB radiation. [Staniforth et al., Carcinogenesis 2006 27(9):1803-1811].

Tartaric acid is known to increase the stool softness and to decrease the intestinal transit time (Spiller et al., British Journal of Nutrition (2003), 90, 803-807).

Consequently, it would be desirable to have available a food product with caffeic acid and/or tartaric acid to produce the benefits described above. However, simply supplementing a foodstuff with caffeic acid and/or tartaric acid might not be ideal, since these compounds might loose activity with time.

It was hence the object of the present invention to prepare a foodstuff that can provide a subject with caffeic acid/ and or tartaric acid that is storage stable and easy to produce.

The present inventors could achieve this object by providing a food composition that allows to produce caffeic acid/ and or tartaric acid *in situ* from a precursor, caftaric acid and/or derivatives thereof.

Caftaric acid is a compound with the following formula 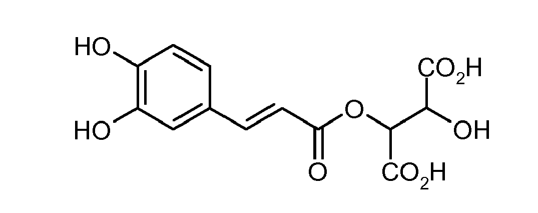 and its derivatives include compounds which exhibit at least one C₁-C₃ alkylation of an OH-group and/or a CO₂H-group For example both phenolic OH-groups may be alkylated. Alternatively and/or additionally both carboxyl groups may be transformed into the corresponding alkylesters. In one embodiment all OH-groups and all CO₂H-groups are alkylated.

Typical derivatives are compounds in which both phenolic OH-groups are methylated, and/or compounds in which both carboxyl groups are methylated.

Further typical derivatives include compounds with the following formula 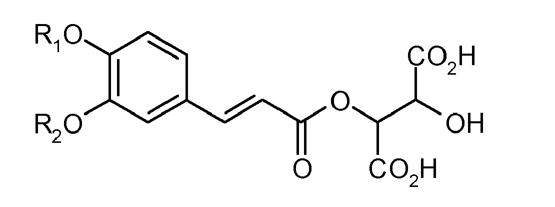 wherein R₁ and/or R₂ are selected from the group consisting of H; CH₃; aryl, such as phenyl, benzyl, tolyl, o-xylylalkyl; C₁-C₃-acyl, amino acids, monosaccharides. R₁ and/or R₂ may be identical or may differ from each other.

One caftaric acid derivative is the following compound:

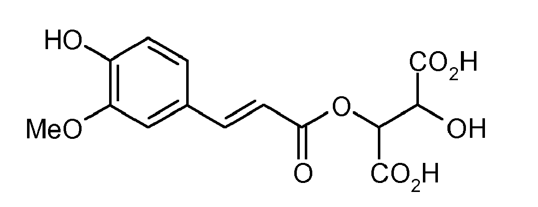

Caftaric acid or its derivatives can then be hydrolysed by a micro-organism and/or an enzyme capable of hydrolysing caftaric acid and/or derivatives thereof. This hydrolysis step will generate tartaric and/or caffeic acid.

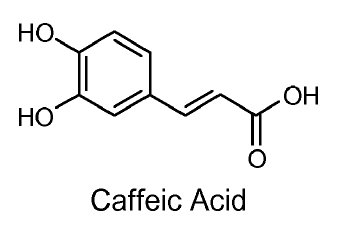

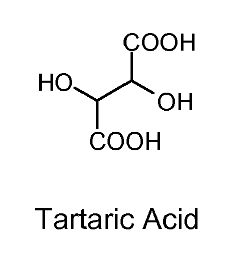

The inventors have surprisingly found that treating an ingredient comprising caftaric acid and/or derivatives thereof with micro-organisms or enzymes capable of hydrolysing caftaric acid and/or derivatives thereof to generate tartaric and/or caffeic acid results for example in improved antioxidant and/or anti-inflammatory properties of the ingredient.

Furthermore, it has been found that this treatment can take place *in vivo* when a human or an animal ingests a composition comprising caftaric acid and/or derivatives thereof in combination with a micro-organism and/or enzyme capable of hydrolysing chlorogenic acids to generate phenolic acids.

Accordingly one embodiment of the present invention is a composition comprising an ingredient containing caftaric acid and/or derivatives thereof, and a micro-organism and/or an enzyme capable of hydrolysing caftaric acid and/or derivatives thereof to generate tartaric and/or caffeic acid.

In a preferred embodiment the ingredient is enriched in caftaric acid and/or derivatives thereof. For example, the ingredient and/or the composition may comprise caftaric acid and/or derivatives thereof in an amount in the range of 0,001-99,99weight-% of dry weight, preferably 0,1-50 weight-% of dry weight, most preferred 0.1-10 weight-% of dry weight. The ingredient and/or the composition may comprise the micro-organism and/or the enzyme capable of hydrolysing caftaric acid and/or derivatives thereof to generate tartaric and/or caffeic acid in an amount in the range of 0,001-99,99 weight-% of dry weight, preferably 0,1-50 weight-% of dry weight, most preferred 0.1-10 weight-% of dry weight.

For example the composition of the present invention may comprise an ingredient containing caftaric acid and/or derivatives thereof and another ingredient comprising a micro-organism and/or an enzyme capable of hydrolysing caftaric acid and/or derivatives thereof to generate tartaric and/or caffeic acid.

The ingredient containing caftaric acid and/or derivatives thereof may be any ingredient that contains caftaric acid and/or derivatives thereof, either naturally or in added form, but is preferably a natural foodstuff such as lettuce, chicory, dandelion, chicory, grape, grape pomace; or combinations or extracts thereof.

The ingredient to be mixed with the ingredient containing caftaric acid and/or derivatives thereof comprises a micro-organism and/or an enzyme capable of hydrolysing caftaric acid and/or derivatives thereof to generate tartaric and/or caffeic acid.

These two ingredients may be mixed briefly prior to consumption or may be provided as a ready-to-consume composition.

The individual ingredients may comprise any kind of edible compound. Typical ingredients for food compositions, in particular drinks, are well known in the art, e.g. milk, cream, coffee whiteners, and coffee creamers. Alternatively, ingredients could also be a salad dressing or parts thereof, for example. Such compounds are used by consumers to modify e.g. the aroma, appearance and texture of a composition. The ingredients may be in liquid or dry form, e.g. as powders, that are dissolved and/or suspended in a drink.

The composition and/or ingredients of the invention should be formulated such that the micro-organism and/or enzyme will not ferment or react with the composition during storage. This may be achieved e.g. by formulating the composition as a dry powder, and/or by encapsulating the micro-organism and/or enzyme so that the micro-organisms and/or enzyme will only be released when the composition is mixed with at least one other ingredient or during digestion.

Consequently, to make sure that the micro-organisms and/or the enzyme act as little as possible on the caftaric acid prior to consumption, the composition may be in the form of a powder, for example the micro-organism and/or enzyme may be present in a dry powdered form, e.g. as a freeze dried powder. The micro-organism and/or enzyme may be encapsulated.

The composition of the invention may further comprise further ingredient suitable for inclusion in a food composition. Usual ingredients may e.g. be sugars, artificial sweeteners, emulsifiers, stabilisers, thickeners, flowing agents, colours, flavours, aromas, and the like. Suitable artificial sweeteners include saccharin, cyclamates, acetosulfame, L-aspartyl based sweeteners such as aspartame, and mixtures of these. Suitable emulsifiers include monoglycerides, diglycerides, lecithin, diacetyl tartaric acid esters of mono-diglycerides, emulsifying starches, and mixtures thereof.

Suitable stabilisers include dipotassium phosphate and sodium citrate. A suitable flowing agent is sodium silica aluminate. In one embodiment the composition comprises milk protein and/or vegetable protein. In a further embodiment the composition comprises milk fat and/or vegetable fat.

The composition of the present invention may comprise a protein source, a carbohydrate source and/or a lipid source. If the composition is intended to be used as a full meal or as a meal replacement it comprises preferably a protein source, a carbohydrate source and a lipid source, so that the nutritional requirements of the subject to be treated are satisfied.

The protein source for example may comprise milk protein and/or vegetable protein, the lipid source may comprise milk fat and/or vegetable fat, and/or the carbohydrate source may comprise milk and/or vegetable carbohydrates, to ensure an optimal nutritional value.

Further, the composition may contain an organic or inorganic carrier material suitable for oral or enteral administration as well as vitamins, minerals trace elements and other micronutrients, for example in accordance with the recommendations of Government bodies such as the USRDA.

In one embodiment the invention relates to a beverage powder comprising: a) a dried water-soluble composition comprising hydrolysing caftaric acid and/or derivatives thereof extract; and b) a micro-organism and/or an enzyme capable of hydrolysing caftaric acid and/or derivatives thereof to generate tartaric and/or caffeic acid.

A beverage powder according to the invention is a powder to be used for the preparation of a beverage by dissolving or suspending the powder in a liquid, e.g. water or milk.

Microorganisms capable of hydrolysing caftaric acid and/or derivatives thereof may be selected from yeasts, fungi or bacteria. Suitable microorganisms may e.g. be an *Aspergillus;* such as e.g. *Aspergillus oryzae, a Lactobacillus*, such as e.g. *L. johnsonii* (CNCM I-1225); a *Bifidobacterium*, such as e.g. *B. lactis* (CNCM I-3446), or a yeast such as e.g. *Saccharomyces cerevisiae*.

Preferred micro-organisms capable of hydrolysing caftaric acid and/or derivatives thereof to generate tartaric and/or caffeic acid are probiotic micro-organisms, preferably lactic acid bacteria, for example *L. johnsonii, B. longum*, and *B. lactis,* even more preferred *Lactobacillus johnsonii* La1 (CNCM 1-1225), *B. longum* BB 536, and *B. lactis* BB12.
*B. longum* BB 536 is commercially available from Morinaga Nutritional Foods, Inc.
*B. lactis* BB12 is commercially available, e.g., from Chr. Hansen, DK-2970 Horsholm.

In one embodiment the micro-organisms may be used in a non-replicating form.

Suitable enzymes that can be used in the framework of the present invention include e.g. esterases, e.g. a chlorogenate esterase derived from *Aspergillus japonicus*. (Commercially available from Kikkoman, Japan), Tannase from *Aspergillus oryzae* (EC 3.1.1.20) (commercially available from Kikkoman, Japan); and Palatase 20000L (EC 3.1.1.3) (commercially available from Novozymes A/S, Denmark). The enzyme may be present as a purified enzyme or e.g. in the form of a cell lysate of a microorganism. Suitable cells may e.g. be cells of the microroganisms mentioned above. Suitable methods for producing cell lysate are known in the art.

Consequently, in one embodiment of the present invention the enzyme capable of hydrolysing caftaric acid to generate tartaric and/or caffeic acid is selected from the group consisting of esterases, such as chlorogenate esterase, tannase and/or feruloyl esterase

The microorganism and/or enzyme should be present in an amount sufficient for hydrolysing a substantial amount of caftaric acid to generate tartaric and/or caffeic acid during digestion. The amount of microorganism and/or enzyme needed may e.g. be determined by those skilled in the art, for example dependent on the subject to be treated or on the speed by which the tartaric and/or caffeic acid should be liberated. Preferably at least 20%, such as at least 30%, at least 50%, or at least 75% of caftaric acid present in the composition is hydrolysed prior to and/or during consumption.

The products of the invention may be used to enhance antioxidant capacity *in vivo* in a human or animal consuming a beverage prepared from the products of the invention, by increasing the Nrf2-mediated gene expression pathway i.e inducing detoxifying enzymes such as gluthathione-S-transferase (GST). An Increased activity of Nrf2 associated genes has been reported to enhance detoxification and to stimulate the endogenous defence against oxidative stress.

The composition of the invention may be used for example to decrease inflammation, e.g. by reducing the prostaglandin E2 level.

Many health problems and disorders, for example age related disorders, are related to oxidative stress and inflammation. The products of the invention may be used to treat or prevent such problems or disorders in a human or animal consuming a beverage prepared from the products of the invention. Relevant problems and disorder are e.g. skin disorders, e.g. photo-damage caused by UV-radiation, atopic dermatitis, eczema, scaling, itching, allergic symptoms; brain disorders; inflammation; obesity; and cancer, e.g. skin cancer and lung cancer.

The composition of the present invention may further be used as anti-diabetic agents, e.g. by reducing blood glucose levels, and/or increasing blood levels of leptin, insulin and/or c-peptide; as bone remodelling agents, e.g. by increasing bone mineral density, e.g. by increasing serum levels of estrogen and/or progesterone and/or alkaline phosphatase activity; as anti-metastatic agents, e.g. with anti-angiogenic effect.

Consequently, one embodiment of the present invention is the use of a composition as described herein for the preparation of a composition to treat or prevent inflammatory disorders, in particular linked to bacterial and/or viral infection; to enhance antioxidant capacity in a human or animal; to treat or prevent oxidative stress related disorders; to treat or prevent metabolic disorders, such as diabetes; to treat or prevent disorders related to bone mineral density; or to treat or prevent cancer.

The composition of the present invention may be intended for oral administration, preferably as food product or drink, or for parenteral application, such as a skin care product.

The present invention also relates to a use of a micro-organism and/or an enzyme capable of hydrolysing caftaric acid and/or derivatives thereof to generate tartaric and/or caffeic acid in a composition containing caftaric acid and/or derivatives thereof.

The micro-organism and/or enzyme used may be the same as described above. For example the micro-organism capable of hydrolysing caftaric acid and/or derivatives thereof to generate tartaric and/or caffeic acid may be a probiotic micro-organism, preferably a lactic acid bacteria, even more preferred *Lactobacillus johnsonii* La1; and/or the enzyme capable of hydrolysing caftaric acid and/or derivatives thereof to generate tartaric and/or caffeic acid may be selected from the group consisting of esterases, such as chlorogenate esterase, tannase and/or feruloyl esterase. The composition - as above - may be a food composition and may further comprise a protein source, a carbohydrate source and/or a lipid source, wherein, preferably, the protein source comprises milk protein and/or vegetable protein, the lipid source comprises milk fat and/or vegetable fat, and/or the carbohydrate source comprises milk and/or vegetable carbohydrates.

In one embodiment the invention relates to a kit for preparing a food product, for example a beverage, comprising at least two parts: a) a first part comprising a caftaric acid and/or derivatives thereof containing food ingredient; and b) a second part comprising a micro-organism and/or an enzyme capable of hydrolysing caftaric acid and/or derivatives thereof to generate tartaric and/or caffeic acid. The two parts may be sold together for the preparation of a food product, for example a beverage, but may be physically separated in the packing of the product. The final food product to be consumed may be prepared by mixing the two parts shortly before consumption. If one or both parts are in a liquid form they may be mixed directly, optionally further liquid, e.g. water or milk, may be added. The two parts may also be mixed by dissolving or suspending them in a liquid, e.g. water or milk. When liquid is used this may be hot or cold. If hot liquid is used, it may preferably have a temperature which is not so high as to inactivate the micro-organism and/or enzyme before ingestion of the beverage.

The first part of the kit may comprise a food product or an extract containing caftaric acid and/or derivatives thereof. In a preferred embodiment the first part is in a dry form, e.g. in the form of a powder. The first part may also be in a liquid form. The first part may additionally comprise any other suitable ingredient, e.g., aroma additives, stabilisers, salts, and/or sweeteners. The first part may be packed in any suitable way, e.g. in a sachet, bottle or can.

The second part comprises a micro-organism and/or an enzyme capable of hydrolysing caftaric acid and/or derivatives thereof to generate tartaric and/or caffeic acid. This part may preferably be in the form of a composition to be mixed with the first part. It may be in dry form, e.g. as a powder, or in liquid form, and may be packed in any suitable way, e.g. in a sachet, bottle or can. It should be formulated such that the micro-organism and/or enzyme will not ferment or react with other ingredients during storage. This may be achieved e.g. by formulating the composition as a dry powder, and/or by encapsulating the micro-organism and/or enzyme so that the micro-organisms and/or enzyme will only be released when the composition is mixed with coffee extract or during digestion.

The at least two parts may be packed together in any suitable way. They may e.g. be packed in a combined container wherein the parts are kept physically separated during storage and mixed when the container is opened, or they may be packed in separate containers which are sold together for the preparation of a beverage.

Those skilled in the art will understand that they can freely combine all features of the present invention described herein, without departing from the scope of the invention as disclosed. In particular, features described for the uses of the present invention may be applied to the composition and/or the kit of the present invention and vice versa.

Further advantages and features of the present invention are apparent from the following Examples and Figures.
Figure 1 shows the hydrolysis of caftaric acid (◆) into caffeic acid (▲) with chlorogenate esterase.
Figure 2 shows the hydrolysis of caftaric acid (◆) with a spray-dried preparation of *L. johnsonii* into caffeic acid (▲).
Figure 3 shows the hydrolysis of caftaric acid in a TIM model with (■) and without (□) La1.

### Examples:

### Hydrolysis of caftaric acid with chlorogenate esterase

A solution of chlorogenate esterase (0.8 mg, 24 U/g, from Kikkoman Japan) in 100 µl phosphate buffer (50 mM, pH 7.0) was added to a solution of caftaric acid (0.37 mg) in 100 µl phosphate buffer (50 mM, pH 7.0). The mixture was then incubated at 37°C for 4 h. After reaction time, the enzymatic activity was stopped by heat treatment (5 min, 90°C) and the mixture was centrifuge (microcon YM10, 30 min, 14000g). The supernatant was then analysed by HPLC. A reaction control was run in parallel under the same reaction conditions but without enzyme.

### Hydrolysis of caftaric acid with L. johnsonii (La1) fresh cells

Cells of *L. johnsonii* (CNCM I-1225) were grown (7.0 E08 cfu/ml) and centrifuged (5000 g, 10 min), the pellets were resuspended in phosphate buffer (50 mM, pH 7.0) at a concentration of 0.61 g/ml. To 100 µl of this cells solution, 100 µl of a solution of caftaric acid (12 mM) was added and the mixture was incubated at 37°C. Samples were withdrawn at different reaction times, centrifuged (3000 g, 5 min) and filtered through 0.45 µm pore size syringe filters (Millipore SLHA 025 BS) and analysed by HPLC.

A reaction control was run in parallel under the same reaction conditions but without bacteria.

Hydrolysis of caftaric acid with La1 extract (lysed cells)

Cells of *L. johnsonii* (CNCM I-1225) were grown (7.0 E08 cfu/ml) and centrifuged (5000 g, 10 min), the pellets were resuspended in phosphate buffer (50 mM, pH 7.0) at a concentration of 0.61 g/ml. The cells were then lysed using the glass-beads method. 600 µl of cells preparation were put into a Mini-Beadbeater for 1 min of intense shaking, cooled in ice, and put another 1 min in the Mini-Beadbeater. The crude cell extract (100 µl) was then added to 100 µl of a solution of caftaric acid (12 mM, phosphate buffer 50 mM, pH 7.0) and the mixture was incubated at 37°C. Samples were withdrawn at different reaction times, centrifuged (3000g, 5 min), filtered through 0.45 µm pore size syringe filters (Millipore SLHA 025 BS) and analysed by HPLC.

### Hydrolysis of caftaric acid with a spray-dried preparation of La1

10 mg of a spray-dried preparation of *L. johnsonii* (3.3 E09 cfu/g) were dissolved in 100 µl of phosphate buffer (50 mM, pH 7.0). To this solution, 100 µl of a caftaric acid solution (12 mM, phosphate buffer 50 mM, pH 7.0) were added. The mixture was then incubated at 37°C and samples were withdrawn at different reaction times. After centrifugation (3000 g, 5 min) and filtration (0.45 µm pore size syringe filters, Millipore SLHA 025 BS) the samples were analysed by HPLC.

### HPLC Analysis

HPLC-DAD analysis of caftaric acid and hydrolysis products was performed on a Agilent 1100 system equipped with a Atlantis C18 reverse-phase column (4.6 x 100 mm, particle size 3 µm) and a diode array detector. The column was equilibrated with water containing 0.1 % formic acid. After injection, a linear gradient to a final solvent composition of 55 % water and 45 % acetonitrile (containing 0.1 % formic acid) was run within 12 min at a flow rate of 1 ml/min. Caftaric acid and caffeic acid were monitored by UV at 320 nm and were quantified using standard calibration curves.

### Gastric small-intestinal model (TIM)

The TNO gastric small-intestinal model, TIM-1, comprises four connected compartments that represent the stomach, duodenum, jejunum and ileum, respectively. Each compartment consists of a glass outer wall with a flexible inner wall. The flexible wall is surrounded by water at 37°C to squeeze the walls, which ensures mixing of the food with the secreted enzymes by peristaltic movements in the gastro-intestinal tract.

The experiments in the model were performed under average physiological conditions of the gastro-intestinal tract. During the experiments, the temperature was kept at 37°C and salivary, gastric, biliary, and pancreatic secretions were simulated. The digestion process in the model was monitored for 6 h. During the first 3.5 h, the gastric content was gradually delivered into the small intestine "pyloric valve". At the end of the experiment, approximately 80 % of the small-intestine content was gradually delivered into the "large intestine" via the ileocaecal valve. Gastric pH gradually decreased from 6.5 to 2.0 in approximately 5 h by the secretion of 1 M HCI; the pH of the small intestinal contents was maintained at 6.5 in the duodenum, 6.8 in the jejunum and 7.2 in the ileum. Products of digestion and water were absorbed from the jejunal and ileal compartments by pumping dialysis liquid through hollow fiber membranes with a molecular weight cut-off of approximately 5 000 Dalton.

### Simulation of caftaric acid digestion

3 g of a spray-dried preparation of La1 (3.3 E9 cfu/g) were dissolved in 295 ml acetate buffer (20 mM, pH 6.5). After addition of 5 ml start residue (pepsin and lipase enzyme solution) the solution was injected into the gastric compartment of TIM. 10 ml of acetate buffer containing 343 mg caftaric acid was then injected by a syringe into the gastric compartment 15 min after starting digestion simulation. During digestion, total dialysate was collected for 0-2, 2-4 and 4-6 h after passage through the semipermeable hollow-fibre membranes connected to the jejunal and ileal compartments. Total ileal delivery was collected for 0-2; 2-4 and 4-6 h. Aliquots (1 ml) were taken from gastric compartment directly after addition of caftaric acid and at time point

1 h. After 6 h, the residues from the compartments of the stomach, duodenum, jejunum and ileum were collected to allow calculating the mass balance of caftaric acid. All samples were passed through a 0.45 µm pore size syringe filters (Millipore SLHA 025 BS) and analysed by HPLC. As a control the same experiment was performed without any addition of La1 bacteria.

### In Vitro tests

### Antioxidant responsive Element (ARE) luciferase assay

The pGL-8xARE which contains eight copies of the ARE present in rat glutathione-S-transferase A2 (GSTA2) along with the pcDNA3.1 plasmid containing the neomycin selectable marker was stably transfected into human MCF7 cells (Wang et al., Cancer Res. 66, 10983-10994, 2006). ARE (antioxidant-responsive element) is the binding site of the transcription factor Nrf2 which regulates the genes involved in detoxification and endogenous defense against oxidative stress. The plasmid pGL-8xARE contains a luciferase gene downstream of the eight Nrf2 binding sites that allows monitoring Nrf2 activity. For treatment with chicoric acid and related compounds, the AREc 32 cells were seeded 96-well microtiter plates in DMEM growth medium. After treatment of 24 h with the different preparations, firefly luciferase activity was determined.

### Tested bacteria

| Bacteria | Culture Media |
|---|---|
| *Lactobacillus rhamnosus* GG (NCC 4007) | MRS |
| *Lactobacillus johnsonii* La1 (CNCM 1-1225) | MRS |
| *Lactobacillus paracasei* ST11 (NCC 2461) | MRS + Cysteine |
| *Bifidobacterium longum* BB 536 (ATCC BAA-999) | MRS + Cysteine |
| *Bifidobacterium lactis* BB12 (CNCM 1-3446) | MRS |
| *Streptococcus thermophilus* TH4 (NCC 2496) | HJL |

Results: *L. johnsonii* (La1), *B. longum* BB 536, and *B. lactis* BB12 were particularly well able to hydrolyse caftaric acid. The best results in terms of reaction rate and reaction yield were obtained with *L. johnsonii* La1.

### Tested enzymes

| Enzyme | Supplier |
|---|---|
| Chlorogenate esterase | Kikkoman, Japan |
| Feruloyl esterase | Novozymes |
| Porcine liver esterase | Sigma E-3019 |
| Hog liver esterase immobilised on Eupergit C | Fluka 46064 |
| Esterase from *Saccharomyces cerevisiae* | Fluka 46071 |

Results: chlorogenate and feruloyl esterases were particularly well able to hydrolyse caftaric acid into caffeic and tartaric acids

### Results

Hydrolysis of caftaric acid with chlorogenate esterase

**Table 1: Hydrolysis of caftaric acid into caffeic acid by chlorogenate esterase**

| | | | | | | |
|---|---|---|---|---|---|---|
| Time (min) | 0 | 15 | 30 | 60 | 120 | 240 |
| Caftaric Acid | 99% | 82% | 64% | 43% | 19% | 2% |
| Caffeic Acid | 0% | 18% | 26% | 57% | 81% | 98% |

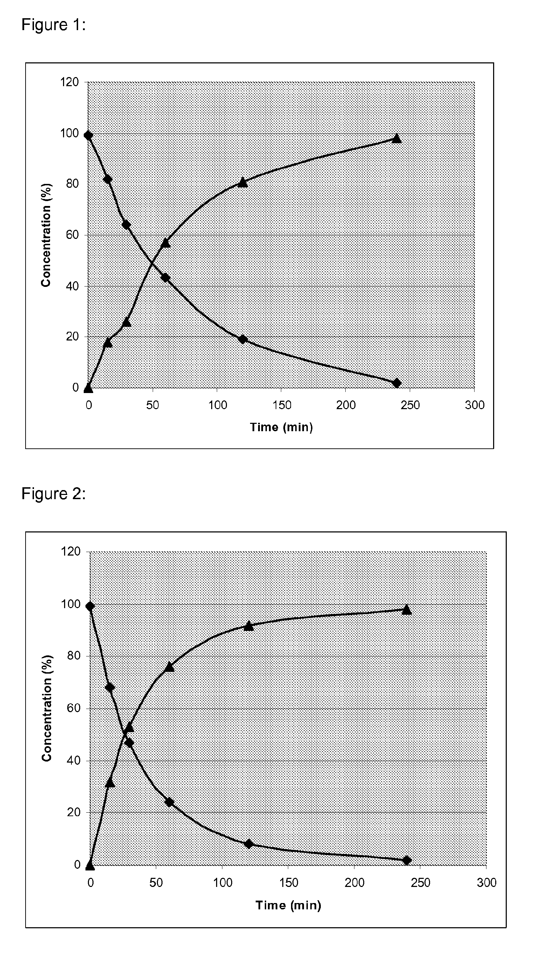

The results are summarised in figure 1.

### Hydrolysis of caftaric acid with La1 fresh cells

After 4h reaction time all caftaric acid was transformed into caffeic acid as analysed by HPLC.

### Hydrolysis of caftaric acid with La1 extract (lysed cells)

As compared to whole cells, the hydrolysis of chicoric acid with lysed cells resulted in an increase of the reaction rate. Indeed, after only 2h all caftaric acid acid was transformed into caffeic acid.

### Hydrolysis of chicoric acid with a spray-dried preparation of La1

**Table 2: hydrolysis of caftaric acid into caffeic acid by a spray-dried preparation of La1**

| | 0 min | 15 min | 30 min | 60 min | 120 min | 240 min |
|---|---|---|---|---|---|---|
| Caftaric Acid | 99 | 68 | 47 | 24 | 8 | 2 |
| Caffeic Acid | 0 | 32 | 53 | 76 | 92 | 98 |

The results are summarized in figure 2.

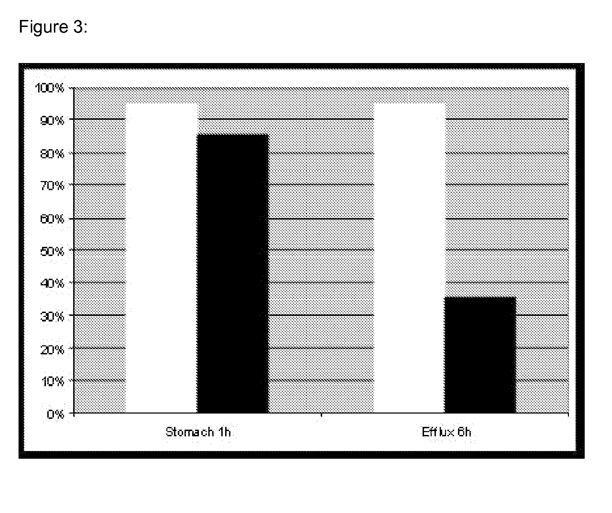

The results of the hydrolysis of caftaric acid in a TIM-Model are summarised in table 3 and figure 3.

**Table 3:**

| | **Caftaric acid** | **Caffeic acid** | **Hydrolysis** |
|---|---|---|---|
| **A15** | | | 0% |
| **A30** | 4364 | 558 | 11.34% |
| **A60** | 3784 | 641 | 14.49% |
| | | | |
| **E2** | 174 | 54 | 23.68% |
| **E4** | 92 | 130 | 58.56% |
| **E6** | 15 | 27 | 64.29% |
| | | | |
| **CD2** | 636 | 128 | 16.75% |
| **CD4** | 343 | 181 | 34.54% |
| **CD6** | 46 | 33 | 41.77% |
| | | | |
| **DD2** | 115 | 25 | 17.86% |
| **DD4** | 122 | 72 | 37.11% |
| **DD6** | 15 | 18 | 54.55% |

### Antioxidant Responsive Element (ARE) luciferase assay

Human breast cancer cells (AREc32) stably transfected with several copies of the rat GSTA2-ARE reporter construct was used to demonstrate the activation of Nrf2-ARE pathway by caftaric acid and related compounds. Caftaric acid not hydrolysed and caftaric acid hydrolysed with La1 (50% and 100%) produced a positive dose-dependent response in Nrf2-luciferase reporter activity (see tables 4, 5) exhibiting a saturation inactivation response at higher doses.

**Table 4: AREc reporter cell line treated with caftaric acid**

| µg/ml | F/C | stdv |
|---|---|---|
| 125 | 2796 | 212 |
| 250 | 5821 | 1900 |
| 500 | 5182 | 680 |
| 750 | 25792 | 5224 |
| 1000 | 4777 | 349 |
| 1200 | 29043 | 8238 |

**Table 5: AREc reporter cell line treated with hydrolysed caftaric acid by La1: (A) 50% hydrolysis, (B) 100% hydrolysis**

| µg/ml | F/C A | F/C B | Stdv-A | Stdv- B |
|---|---|---|---|---|
| 50 | 3809 | 3878 | 2326 | 1969 |
| 75 | 6896 | 4241 | 3553 | 779 |
| 100 | 9427 | 11332 | 3757 | 4410 |
| 125 | 10564 | 12040 | 622 | 3453 |
| 150 | 11588 | 16845 | 8650 | 1093 |
| 200 | 1479 | 347 | 2105 | 120 |

## Claims

**1.** A composition comprising an ingredient containing caftaric acid and/or derivatives thereof, and a micro-organism and/or an enzyme capable of hydrolysing caftaric acid and/or derivatives thereof to generate tartaric and/or caffeic acid.

**2.** The composition of claim 1, further comprising a protein source, a carbohydrate source and/or a lipid source.

**3.** The composition of claim 2, wherein the protein source comprises milk protein and/or vegetable protein, the lipid source comprises milk fat and/or vegetable fat, and/or the carbohydrate source comprises milk and/or vegetable carbohydrates.

**4.** The composition of any of claims 1-3 wherein the composition is in the form of a powder.

**5.** The composition of any of claims 1-4 wherein the micro-organism capable of hydrolysing caftaric acid and/or derivatives thereof to generate tartaric and/or caffeic acid is a probiotic micro-organism, preferably a lactic acid bacteria, for example *L. johnsonii, B. longum,* and *B. lactis*, even more preferred *Lactobacillus johnsonii* La1 (CNCM I-1225), *B. longum* BB 536, and *B. lactis* BB12.

**6.** The composition of any of claims 1-5 wherein the enzyme capable of hydrolysing caftaric acid to generate tartaric and/or caffeic acid is selected from the group consisting of esterases, such as chlorogenate esterase, tannase and/or feruloyl esterase

**6.** The composition of any of claims 1-5, wherein the caftaric acid containing ingredient is a natural foodstuff such as lettuce, chicory, lettuce, dandelion, chicory, grape, grape pomace; or combinations or extracts thereof.

**7.** Use of a composition in accordance with one of claims 1-6 for the preparation of a composition to treat or prevent inflammatory disorders" in particular linked to bacterial and/or viral infection; to enhance antioxidant capacity in a human or animal; to treat or prevent oxidative stress related disorders; to treat or prevent metabolic disorders, such as diabetes; to treat or prevent disorders related to bone mineral density; or to treat or prevent cancer.

**8.** Use in accordance with claim 7, wherein the composition is intended for oral administration, preferably as food product or drink, or for parenteral application, such as a skin care product.

**9.** Use of a micro-organism and/or an enzyme capable of hydrolysing caftaric acid and/or derivatives thereof to generate tartaric and/or caffeic acid in a composition containing caftaric acid and/or derivatives thereof.

**10.** Use in accordance with one of claim 9, wherein the micro-organism capable of hydrolysing caftaric acid and/or derivatives thereof to generate tartaric and/or caffeic acid is a micro-organism, preferably a lactic acid bacteria, preferably a probiotic, even more preferred *Lactobacillus johnsonii* La1; and/or the enzyme capable of hydrolysing caftaric acid and/or derivatives thereof to generate tartaric and/or caffeic acid is selected from the group consisting of esterases, such as chlorogenate esterase, tannase and/or feruloyl esterase.

**11.** Use in accordance with claim one of claims 9-10, wherein the composition is a food composition and further comprises a protein source, a carbohydrate source and/or a lipid source, wherein, preferably, the protein source comprises milk protein and/or vegetable protein, the lipid source comprises milk fat and/or vegetable fat, and/or the carbohydrate source comprises milk and/or vegetable carbohydrates.

**12.** A kit for preparing a food product, comprising at least two parts:
a) a first part comprising a caftaric acid and/or derivatives thereof containing food ingredient; and
b) a second part comprising a micro-organism and/or an enzyme capable of hydrolysing caftaric acid and/or derivatives thereof to generate tartaric and/or caffeic acid.

**13.** The kit of claim 12 wherein the micro-organism capable of hydrolysing caftaric acid and/or derivatives thereof to generate tartaric and/or caffeic acid is a micro-organism, preferably a probiotic preferably a lactic acid bacteria, even more preferred *Lactobacillus johnsonii* La1; and/or the enzyme capable of hydrolysing caftaric acid and/or derivatives thereof to generate tartaric and/or caffeic acid is selected from the group consisting of esterases, such as chlorogenate esterase, tannase and/or feruloyl esterase.

**14.** The kit of claim 12 or 13 wherein the second part comprises a protein source, a carbohydrate source and/or a lipid source, wherein, preferably, the protein source comprises milk protein and/or vegetable protein, the lipid source comprises milk fat and/or vegetable fat, and/or the carbohydrate source comprises milk and/or vegetable carbohydrates.
